# EUROPEAN PATENT SPECIFICATION

(11) **EP 2 727 559 B1**
(45) Date of publication and mention of the grant of the patent: **22.05.2024**
(21) Application number: 12804332.0
(22) Date of filing: 20.06.2012
(51) Int. Cl.: A61F 2/28, A61L 27/32, A61L 27/06, A61L 27/04

(54) **SPINOUS METAL PARTICLE**
DORNIGE METALLPARTIKEL
PARTICULE MÉTALLIQUE ÉPINEUSE

(30) Priority: 29.06.2011 CN 201120223913 U; 29.06.2011 CN 201120223915 U
(43) Date of publication of application: 07.05.2014
(73) Proprietor: Beijing AK Medical Co., Ltd., 102200, Beijing (CN)
(72) Inventor: CAI, Hong, 102200 Beijing (CN); ZHANG, Weiping, 102200 Beijing (CN)
(74) Representative: Müller-Boré & Partner Patentanwälte PartG mbB
(86) International application number: PCT/CN2012/077255
(87) International publication number: WO 2013/000373

(56) References cited:
- WO-A2-01/66044
- CN-A- 1 356 914
- CN-A- 1 426 290
- CN-U- 202 154 766
- CN-U- 202 161 436
- US-A- 5 178 201
- US-A1- 2008 242 529
- US-B1- 7 056 577

## Description

### Technical field of the invention

The disclosure relates to implants for orthopaedic surgery, in particular to a spinous metal granule for supporting and filling the collapse of articular cartilage.

### Background of the invention

The avascular necrosis of femoral head is a common orthopedic disease; and the hip replacement surgery for treating such disease has been very mature in the modern medical field. Whereas, in some early cases, for the relatively slight depression of the articular surface of cartilage caused by the deterioration and defect of cancellous bone of cartilage of femoral head, if the cartilage of femoral head is certainly supported in time and its degenerated and defected bone structure is reconstructed and repaired by some means, the process of necrosis of femoral head can be retarded or even stopped, and accordingly, the hip replacement surgery can be delayed greatly for a patient. There are several similar treatment methods in the modern medical field, such as implanting an autogenous bone or an allogeneic bone to the cartilage, or inlaying a metal support etc.. However, due to the limited sources of the autogenous bone and the high price and biological risk of the allogeneic bone, not every patient needing to fill the bone defect can get a satisfactory treatment. Furthermore, the inlayed metal support is restrained by the thickness of the femoral neck and the implanted support is limited in diameter, so that they cannot be truly filled in the defect cavity of the cartilage of femoral head, and accordingly, the treating effect is also limited.

US 7,056,577 B1 discloses an irregularly shaped implant grain, and the grain includes multiple pores that are continuous through said grain, and openings of said multiple pores and ducts or passages interconnecting at least a portion of said multiple pores have a width of greater than about 50 µm.

WO 01/66044 A2 discloses a shaped particle for use in an array of interlocking particles to repair, replace or augment a bone deficiency, and the shaped particle comprises a center portion and at least four tapered extremities projecting from said center portion, wherein said projections provide for interstitial spaces between adjacent extremities. And the particle in a preferred embodiment has six extremities, and the interstitial spaces between the extremities of one particle accept the extremities of an adjacent particle in an array.

US 5,178,201 B1 discloses a method for producing an implant with an open-celled structure at least partly covering its surface.

### Summary of the invention

The objective of the disclosure is to provide a spinous metal granule for supporting and filling the collapse of cartilage. The spinous metal granule is provided with spinous structure like a urchin or dendrite. During an operation, a certain number of spinous metal granules can be mixed and stirred with the bone, bone granule and bone paste of an autogenous bone or an allogeneic bone to be filled in the bone defect cavity below the depression of cartilage of femoral head as required and may fill the bone defect cavity up in a full filling form after being pressed. Due to its own strength and the embedded inosculation between its external spines and the surrounding bone tissues, the spinous metal granule can play an initial stabilizing role on the implanted fillers to support the depressed part of the cartilage. In the recovery process of a patient, bone cells and capillaries crawl and grow in along with the bone, bone granule, bone paste and the like of the autogenous bone or the allogeneic bone implanted by being stirred and mixed with the spinous metal granule, to finally form a fusion embedded active bone repairing body.

Based on the inventive concept above, a metal granule which can be mixed and stirred with the bone, bone granule, bone paste and the like of the autogenous bone or the allogeneic bone to be filled in the bone defect cavity below the depression of cartilage of femoral head is provided. In the recovery process of a patient, bone cells and capillaries crawl and grow in along with the bone, bone granule, bone paste and the like of the autogenous bone or the allogeneic bone implanted by being stirred and mixed with the metal granule, to finally form a fusion embedded active bone repairing body. The disclosure further provides a spinous metal granule with bone grafting pores. During an operation, a certain number of bone grafting filling metal granules can be mixed and stirred with the bone, bone granule and bone paste of an autogenous bone or an allogeneic bone as required to fill the bone defect and may fill the bone defect cavity up in a full filling form after being pressed. Due to its own strength and the embedded inosculation between its external spines and the surrounding bone tissues, the spinous metal granule plays an initial stabilizing role on the implanted fillers to support the depressed part of the cartilage and partially replaces the autogenous bone or the allogeneic bone filler in a space occupying form at the same time. In the recovery process of a patient, bone cells and capillaries grow into the bone grafting filling metal granule to form a fusion embedded biologically-active bone repairing body.

To achieve the objective above, the disclosure adopts the following design solutions:
The spinous metal granule has a spherical, cylindrical or polyhedral outline, which is 2 to 15mm in size, and is provided with spinous structure like a urchin or dendrite. When in use, an appropriate number of spinous metal granules are mixed and stirred with the bone, bone granule and bone paste of an autogenous bone or an allogeneic bone to be filled in the bone defect.

Furthermore, the spinous metal granule includes a metal granule and a spinous structure like a urchin or dendrite on the metal granule. When the spinous metal granule is mixed and stirred with the bone, bone granule and bone paste of the autogenous bone or the allogeneic bone to be implanted to the bone defect, its spinous part may be embedded into the bone tissues surrounding the defect cavity to form initial positioning. The spinous structure is only for supporting and embedding, so that its cross section can be of any shape. Furthermore, the spines of each spinous metal granule can be embedded with one another so that the relative positions between the adjacent spinous metal granules and the bone, bone granule and bone paste are locked to realize certain overall strength, which can support the collapsed cartilage.

Furthermore, the spinous metal granule is made of medical metals, including but not limited to titanium, titanium alloy, cobalt alloy, stainless steel, tantalum and magnesium alloy.

Furthermore, the surface of the spinous metal granule is completely or partially applied with hydroxyapatite coatings, which can induce the growth of bone cells.

Furthermore, the method for processing the spinous metal granule includes laser or high-energy electronic beam rapid forming technology, high-temperature sintering, chemical corrosion, electrochemical deposition and the like; and other processing methods further include precision casting, welding, mechanical cutting, electrical discharge machining and the like. The hydroxyapatite coatings are obtained by high-temperature spray or electrochemical deposition.

Furthermore, the spinous metal granule with the bone grafting pores is spherical, cylindrical, polyhedral, spiral or reticulate or the combinations thereof, and the metal granule is 2 to15 mm in outline size and is provided with a spinous structure. When in use, an appropriate number of spinous metal granules with the bone grafting pores are mixed and stirred with the bone, bone granule and bone paste of an autogenous bone or an allogeneic bone to be filled in the bone defect part.

Furthermore, bone grafting pores for accommodating implanted broken bone granules or bone paste are formed on the metal granule of the spinous metal granule with the bone grafting pores, and are extended inwards from the outer surface of the metal granule, wherein the aperture of the bone grafting pores is 1 to 10mm and the bone grafting pores are penetrated with one another.

Furthermore, micropores which are communicated with one another are extended inwards from the outer surface of the metal granule and the inner surfaces of the bone grafting pores of the spinous metal granule with the bone grafting pores, and the aperture of the micropores is 50 to 900 microns.

Furthermore, the spinous metal granule with the bone grafting pores is made of medical metals, including but not limited to titanium, titanium alloy, cobalt alloy, stainless steel, tantalum and magnesium alloy.

Furthermore, the surface of the spinous metal granule with the bone grafting pores is completely or partially applied with hydroxyapatite coatings, which can induce the growth of bone cells.

Furthermore, the method for processing the spinous metal granule with the bone grafting pores includes: forging, casting, mechanical cutting and drilling, electrical discharging machining and the like; and other processing methods further include laser or high-energy electronic beam rapid forming technology, high temperature sintering, chemical corrosion, electrochemical deposition and the like. Particularly, through such technologies as the laser or high-energy electronic beam rapid forming technology, high temperature sintering, chemical corrosion and electrochemical deposition, the spinous structure of the spinous metal granule with the bone grafting pores can be manufactured and micropores which are communicated with one another can be further formed in the surface of the metal granule and the inner surfaces of the bone grafting pores. The hydroxyapatite coatings are obtained by high-temperature spray or electrochemical deposition.

In another aspect of the disclosure, a mixed filler for supporting the collapse of cartilage is provided, which includes the spinous metal granule.

Furthermore, the mixed filler further includes auxiliary materials, which are selected from one or more of the bone, bone granule or bone paste of an autogenous bone or an allogeneic bone.

The invention is defined in claim 1, with further embodiments defined in the dependent claims.

The disclosure has the following advantages:
1. The spinous metal granule has a spherical, cylindrical or polyhedral outline, which is 2 to 15mm in size, and is provided with spinous structure like a urchin or dendrite, so as to provide multiple choices of various bone defect filling shapes and sizes.
2. When the spinous metal granule is in use, an appropriate number of spinous metal granules of appropriate size and shape are selected by a doctor to be mixed and stirred with the autogenous or allogeneic broken bone granules or bone paste of a patient as required to form the mixed filler, which is filled in the bone defect and is pressed and tamped to be initially stabilized. In the recovery process after an operation, the surrounding sclerotins and the bone paste and broken granules surrounding the spinous metal granule are fused to grow into a whole to form a relatively stable and biologically active support structure. Meanwhile, such support structure can stimulate the surrounding blood supply to promote the repairing of the damaged cartilage. The spinous metal granule can partially replace the autogenous bone or the allogeneic bone, so as to greatly reduce the economic burden of the patient and overcome the difficulty and defect in the number of the acquired autogenous and the allogeneic bones to certain extent; meanwhile, the spinous structure of the spinous metal granule may be embedded into the bone tissues surrounding the defect cavity to form initial positioning, which may generate a much greater support force on the cartilage than that generated by a traditional bone and bone granule filling method; and the autogenous broken bone granule and bone paste of the patient can be obtained by osteotomy, bone drilling and other operation processes in operation.
3. The spinous metal granule with the bone grafting pores is spherical, cylindrical, polyhedral, spiral or reticulate or the combinations thereof, and is 2 to15 mm in outline size. When in use, the spinous metal granules of various shapes, sizes and numbers can be selected to be mixed and stirred with the bone, bone granule and bone paste of the autogenous bone or the allogeneic bone to be effectively filled in the bone defect parts of various shapes in a full filling form; the spinous structure of the spinous metal granule may be embedded into the bone tissues surrounding the defect cavity to form an initial positioning and partially replace the fillers of the autogenous bone or the allogeneic bone in a space occupying form at the same time.
4. Bone grafting pores for accommodating implanted broken bone granules or bone paste are formed on the metal granule of the spinous metal granule with the bone grafting pores and are extended inwards from the outline of the bone grafting filling metal granule, wherein the aperture of the bone grafting pores is 1 to 10mm and the bone grafting pores are communicated with one another. In the recovery process after the operation, the sclerotins surrounding the defect are fused with the bone paste and broken bone granule in the bone grafting pores to grow into a whole, thereby effectively preventing the bone grafting filling metal granule from being loosened in a long time.
5. Micropores which are communicated with one another are extended inwards from the outer surface of the metal granule and the inner surfaces of the bone grafting pores of the spinous metal granule with the bone grafting pores and are tightly contacted with sclerotins, which facilitates the ingrowth of bone cells and blood vessels, and long-term stability can be obtained. The aperture of the micropores is 50 to 900 microns, and the micropores of such size can promote the bone cells and capillaries to crawl and grow into the bone grafting filling metal granule to finally form a fusion embedded biologically-active bone repairing body.
6. The surface of the spinous metal granule is completely or partially applied with hydroxyapatite coatings, which can induce the growth of bone cells.
7. The spinous metal granule is made of medical metals, including but not limited to titanium, titanium alloy, cobalt alloy, stainless steel, tantalum and magnesium alloy, and the biocompatibility of these metals has been proved by years of orthopedic implantation application at home and abroad.
8. The method for processing the spinous metal granule provided by the disclosure includes: laser or high-energy electronic beam rapid forming technology, high-temperature sintering, chemical corrosion, electrochemical deposition and the like; and other processing methods further include precision casting, welding, mechanical cutting, electrical discharge machining and the like. The hydroxyapatite coatings are obtained by high-temperature spraying or electrochemical deposition.

The method for processing the spinous metal granule by the laser or high-energy electronic beam rapid forming technology is as follows:
1), the 3D data model of the spinous metal granule is constructed in a computer;
2), the 3D data model is layered by professional software to obtain the outline data of a series of single-layer slices;
3), the data of the series of slices is input to the laser or high-energy electronic beam fast forming equipment;
4), medical metal powder, the thickness of which corresponds to the layering height of the 3D data model, is laid in the processing chamber of the laser or high-energy electronic beam fast forming equipment;
5), the medical metal powder is scanned or selectively melt by the laser or high-energy electronic beam under the control of a computer;
6), the steps of laying, scanning and melting the powder are repeated to sinter the materials selected to be melt in each layer into a whole, and
7), after the melting is completed in all layers, the powder not molten is removed to obtain the spinous metal granule in the required shape.

At present, the typical metal fast forming equipment includes: laser EOSINT M270 metal laser sintering system from EOS company of German and EBM A1 and A2 electronic beam melting systems from ARCAM company of Sweden.

Other processing methods above have been the mature technologies well-known in the metal processing industry, thereby needing no further description in the disclosure.

### Brief description of the drawings

Fig. 1 is a diagram showing the spherical structure of a spinous metal granule in one embodiment of the disclosure;
Fig. 2 is a diagram showing the cylindrical structure of a spinous metal granule in one embodiment of the disclosure;
Fig. 3 is a diagram showing the polyhedral structure of a spinous metal granule in one embodiment of the disclosure;
Fig. 4 is a diagram showing the spinous metal granule filled in the cartilage of the femoral head;
Fig. 5 is a diagram showing the spherical structure of metal granule of a spinous metal granule with bone grafting pores in one embodiment of the disclosure;
Fig. 6 is a diagram showing the cylindrical structure of metal granule of a spinous metal granule with bone grafting pores in one embodiment of the disclosure;
Fig. 7 is a diagram showing the spiral structure of metal granule of a spinous metal granule with bone grafting pores in one embodiment of the disclosure;
Fig. 8 is a diagram showing the reticulate structure of metal granule of a spinous metal granule with bone grafting pores in one embodiment of the disclosure;
Fig. 9 is a diagram showing the polyhedral structure of metal granule of a spinous metal granule with bone grafting pores in one embodiment of the disclosure; and
Figs. 10 to 13 are diagrams showing the combined structure of various shapes of a spinous metal granule in embodiments of the disclosure.

### Detailed description of the invention

The embodiments of the disclosure are further explained below in conjunction with the accompanying drawings.

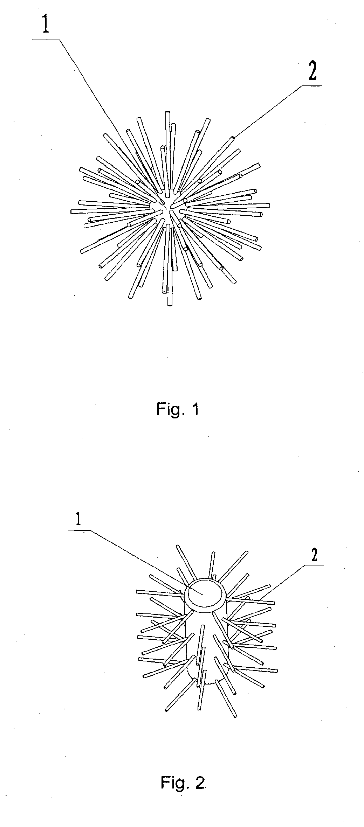

According to one typical embodiment of the disclosure, as shown in Figs. 1, 2 and 3, the spinous metal granule has a spherical, cylindrical or polyhedral outline, which is 2 to 15mm in size, and is provided with spinous structure 2 like a urchin or dendrite. The spinous structure 2 is only used for supporting and embedding, so that its cross section can be of any shape.

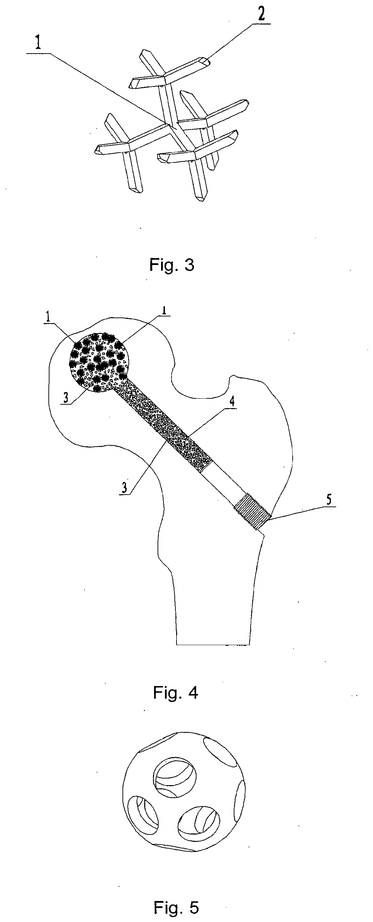

As shown in Fig. 4, during an operation, a channel 4 which can reach the depressed position of the cartilage of femoral head is opened at first; and then the spinous metal granule 1 is mixed and stirred with autogenous or allogeneic broken bone granule and bone paste 3 to form a mixed filler, which is filled at the bone defect and is pressed and tamped. The channel 4 can be filled by the mixed filler formed by stirring and mixing the spinous metal granule 1 with the autogenous or allogeneic broken bone granule and bone paste 3 or only by the autogenous or allogeneic broken bone granule and bone paste 3, and is further blocked by a bone screw 5 when necessary. The admission and direction of the channel 4 are determined by the doctor according to the specific condition of the patient in the operation.

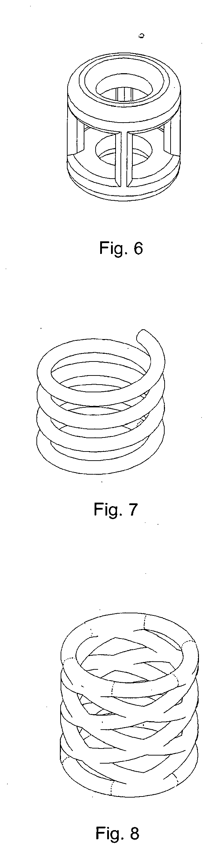

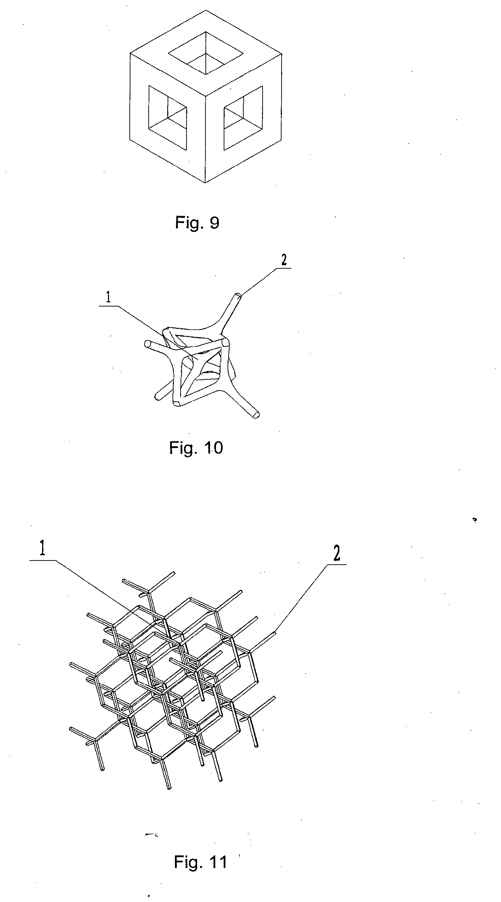

According to one typical embodiment of the disclosure, the spinous metal granule can be formed by the metal granule and the spinous structure 2 like a urchin or dendrite on the metal granule. The metal granule in the spinous metal granule with bone grafting pores can be spherical, cylindrical, polyhedral, spiral or reticulate as shown in Figs. 5 to 12 or the combinations thereof; and the dimension of the outline of the metal granule is 2 to 15mm.

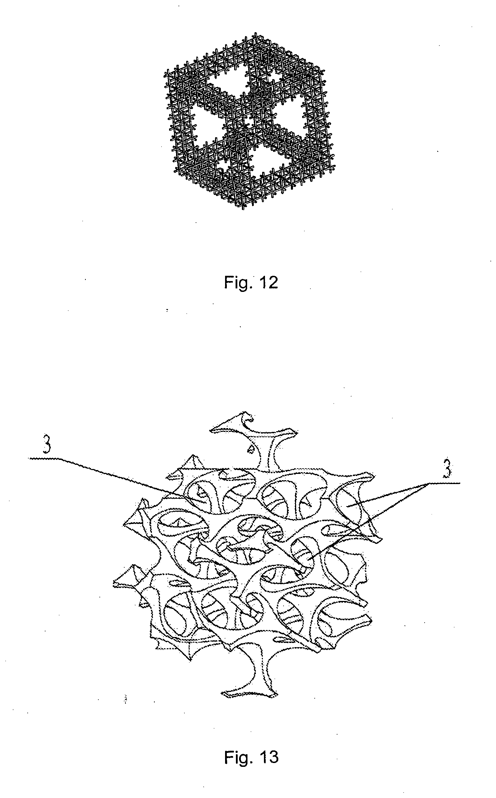

Preferably, bone grafting pores for accommodating implanted broken bone granule or bone paste are formed in the metal granule and are extended inwards from the outer surface of the metal granule, wherein the aperture of the bone grafting pores is 1 to 10mm. Preferably, as shown in Fig. 13, the bone grafting pores are penetrated with one another. Micropores 3 which are communicated with one another are extended inwards from the outer surface of the metal granule and the inner surfaces of the bone grafting pores, and the aperture of the micropores is 50 to 900 microns.

The disclosure further provides a bone grafting filler, which includes the spinous metal granule above. Furthermore, the bone grafting filler further includes auxiliary materials, which are selected from one or more of the bone, bone granule or bone paste of the autogenous bone or the allogeneic bone.

The method for processing the spinous metal granule 1 includes: laser or high-energy electronic beam rapid forming technology, high-temperature sintering, chemical corrosion, electrochemical deposition and the like; and other processing methods further include precision casting, welding, mechanical cutting, electrical discharge machining and the like. The micropores 3 can be processed by such technologies as laser or high-energy electronic beam rapid forming technology, high-temperature sintering, chemical corrosion and electrochemical deposition; and the hydroxyapatite coatings are obtained by high-temperature spraying or electrochemical deposition. The processing methods above are all very mature so as to avoid technical difficulty in the application.

The spinous metal granule is made of medical metals, including but not limited to titanium, titanium alloy, cobalt alloy, stainless steel, tantalum and magnesium alloy; and the metals are specified in the ISO-583 series international standard and their biocompatibility has been proved by years of orthopedic implantation application at home and abroad.

## Claims

1. A spinous metal granule, wherein the spinous metal granule (1) is spherical, cylindrical, polyhedral, spiral or reticulate or combinations thereof, and the metal granule is 2 to 15mm in outline size and is provided with spinous structures (2) like an urchin or a dendrite, **characterized in that** the spinous metal granule comprises bone grafting pores.

2. The spinous metal granule according to claim 1, **characterized in that** micropores (3) which are communicating with one another are extended inwards from the outer surface of the metal granule and the inner surfaces of the bone grafting pores.

3. The spinous metal granule according to claim 1, **characterized in that** hydroxyapatite coatings are applied on all or part of the surface of the spinous metal granule and the inner surfaces of the bone grafting pores.

4. The spinous metal granule according to claim 1, **characterized in that** the spinous metal granule (1) is made of a medical metal, which is selected from one of titanium, titanium alloy, cobalt alloy, stainless steel, tantalum and magnesium alloy.

5. A mixed filler for supporting the collapse of cartilage, **characterized by** comprising a number of spinous metal granules as defined in any one of claims 1 to 4.

6. The mixed filler according to claim 5, **characterized by** further comprising auxiliary materials, which are selected from one or more of bone, bone granule or bone paste of an autogenous bone or an allogeneic bone.

## Patentansprüche

1. Metalldornkörnchen, wobei das Metalldornkörnchen (1) kugelförmig, zylindrisch, polyedrisch, spiralförmig oder netzartig oder eine Kombination davon ist und das Metallkörnchen eine Umrissgröße von 2 bis 15 mm aufweist und mit dornigen Strukturen (2) versehen ist, wie ein Seeigel oder ein Dendrit, **dadurch gekennzeichnet, dass** das Metalldornkörnchen Knochenaufbauporen aufweist.

2. Metalldornkörnchen nach Anspruch 1, **dadurch gekennzeichnet, dass** sich von der Außenfläche des Metallkörnchens und den Innenflächen der Knochenaufbauporen miteinander kommunizierende Mikroporen (3) nach innen erstrecken.

3. Metalldornkörnchen nach Anspruch 1, **dadurch gekennzeichnet, dass** Hydroxylapatitbeschichtungen auf der gesamten oder einem Teil der Oberfläche des Metalldornkörnchens und den Innenflächen der Knochenaufbauporen aufgebracht sind.

4. Metalldornkörnchen nach Anspruch 1, **dadurch gekennzeichnet, dass** das Metalldornkörnchen (1) aus einem medizinischen Metall ist, das aus Titan, Titanlegierung, Kobaltlegierung, Edelstahl, Tantal und Magnesiumlegierung ausgewählt ist.

5. Füllstoffgemisch zur Unterstützung des Knorpelkollapses, **dadurch gekennzeichnet, dass** es eine Anzahl von Metalldornkörnchen gemäß einem der Ansprüche 1 bis 4 umfasst.

6. Füllstoffgemisch gemäß Anspruch 5, **dadurch gekennzeichnet, dass** es weiter Hilfsstoffe umfasst, die aus Knochen, Knochengranulat oder Knochenpaste eines autogenen Knochens oder eines allogenen Knochens ausgewählt sind.

## Revendications

1. Granule métallique épineux, dans lequel le granule métallique épineux (1) est sphérique, cylindrique, polyédrique, en spirale ou réticulé ou des combinaisons de ceux-ci, et le granule métallique est de 2 à 15 mm en taille de contour et est pourvu de structures épineuses (2) comme un oursin ou une dendrite, **caractérisé en ce que** le granule métallique épineux comprend des pores de greffe osseuse.

2. Granule métallique épineux selon la revendication 1, **caractérisé en ce que** des micropores (3) qui communiquent l'un avec l'autre sont étendus vers l'intérieur depuis la surface externe du granule métallique et les surfaces internes des pores de greffe osseuse.

3. Granule métallique épineux selon la revendication 1, **caractérisé en ce que** des enrobages d'hydroxyapatite sont appliqués sur l'ensemble ou une partie de la surface du granule métallique épineux et les surfaces internes des pores de greffe osseuse.

4. Granule métallique épineux selon la revendication 1, **caractérisé en ce que** le granule métallique épineux (1) est réalisé en un métal médical qui est sélectionné parmi un du titane, d'un alliage de titane, d'un alliage de cobalt, de l'acier inoxydable, du tantale et d'un alliage de magnésium.

5. Charge mélangée pour supporter l'effondrement de cartilage, **caractérisée par** le fait de comprendre un certain nombre de granules métalliques épineux selon l'une quelconque des revendications 1 à 4.

6. Charge mélangée selon la revendication 5, **caractérisée par** le fait de comprendre en outre des matériaux auxiliaires qui sont sélectionnés parmi un ou plusieurs d'un os, d'un granule osseux ou d'une pâte osseuse d'un os autogène ou d'un os allogénique.
